# EUROPEAN PATENT SPECIFICATION

(11) **EP 1 976 864 B1**
(45) Date of publication and mention of the grant of the patent: **26.09.2012**
(21) Application number: 06702901.7
(22) Date of filing: 23.01.2006
(51) Int. Cl.: C07K 14/00, C07K 14/50

(54) **NOVEL PEPTIDE AND USE THEREOF**
NEUES PEPTID UND SEINE VERWENDUNG
NOUVEAU PEPTIDE ET SON UTILISATION

(43) Date of publication of application: 08.10.2008
(73) Proprietor: Imagene Co., Ltd., Seoul 151-742 (KR)
(72) Inventor: KIM, Sung-hoon, Seoul 135-536 (KR); HAN, Jung-min, Seoul 150-935 (KR)
(74) Representative: Høiberg A/S
(86) International application number: PCT/KR2006/000252
(87) International publication number: WO 2007/083853

(56) References cited:
- WO-A1-2006/083087
- US-A- 5 981 606
- US-A1- 2005 119 175
- PARK SANG GYU ET AL: "The novel cytokine p43 stimulates dermal fibroblast proliferation and wound repair." THE AMERICAN JOURNAL OF PATHOLOGY FEB 2005 LNKD- PUBMED:15681823, vol. 166, no. 2, February 2005 (2005-02), pages 387-398, XP002592771 ISSN: 0002-9440
- DATABASE GENPEPT [Online] 14 February 1995 XP003015941 Retrieved from NCBI Database accession no. (AAA62202)
- PARK S.G. ET AL.: 'Functional expansion of aminoacyl-tRNA synthetase and their interacting factors: new perspectives on housekeepers' TRENDS BIOCHEM. SCI. vol. 30, no. 10, October 2005, pages 569 - 574, XP005090075

### SEQUENCE LISTING

<110> Imagene Co., Ltd.
<120> Novel peptide and use thereof
<160> 37
<170> KopatentIn 1.71
<210> 1
   <211> 41
   <212> PRT
   <213> Homo sapiens
<220>
   <221> PEPTIDE
   <222> (1)..(41)
   <223> AIMP1-(6-46)
<400> 1
   Ala Val Leu Lys Arg Leu Glu Gln Lys Gly Ala Glu Ala Asp Gln Ile
   1 5 10 15
   Ile Glu Tyr Leu Lys Gln Gln Val Ser Leu Leu Lys Glu Lys Ala Ile
   20 25 30
   Leu Gln Ala Thr Leu Arg Glu Glu Lys
   35 40
<210> 2
   <211> 46
   <212> PRT
   <213> Homo sapiens
<220>
   <221> PEPTIDE
   <222> (1)..(46)
   <223> AIMP-(1-46)
<400> 2
<210> 3
   <211> 53
   <212> PRT
   <213> Homo sapiens
<220>
   <221> PEPTIDE
   <222> (1)..(53)
   <223> AIMP1-(1-53)
<400> 3
   Met Ala Asn Asn Asp Ala Val Leu Lys Arg Leu Glu Gln Lys Gly Ala
   1 5 10 15
   Glu Ala Asp Gln Ile Ile Glu Tyr Leu Lys Gln Gln Val Ser Leu Leu
   20 25 30
   Lys Glu Lys Ala Ile Leu Gln Ala Thr Leu Arg Glu Glu Lys Lys Leu
   35 40 45
   Arg Val Glu Asn Ala
   50
<210> 4
   <211> 187
   <212> PRT
   <213> Homo sapiens
<220>
   <221> PEPTIDE
   <222> (1)..(187)
   <223> AIMP1-(6-192)
<400> 4
   Ala Val Leu Lys Arg Leu Glu Gln Lys Gly Ala Glu Ala Asp Gln Ile
   1 5 10 15
   Ile Glu Tyr Leu Lys Gln Gln Val Ser Leu Leu Lys Glu Lys Ala Ile
   20 25 30
   Leu Gln Ala Thr Leu Arg Glu Glu Lys Lys Leu Arg Val Glu Asn Ala
   35 40 45
   Lys Leu Lys Lys Glu Ile Glu Glu Leu Lys Gln Glu Leu Ile Gln Ala
   50 55 60
   Glu Ile Gln Asn Gly Val Lys Gln Ile Ala Phe Pro Ser Gly Thr Pro
   65 70 75 80
   Leu His Ala Asn Ser Met Val Ser Glu Asn Val Ile Gln Ser Thr Ala
   85 90 95
   Val Thr Thr Val Ser Ser Gly Thr Lys Glu Gln Ile Lys Gly Gly Thr
   100 105 110
   Gly Asp Glu Lys Lys Ala Lys Glu Lys Ile Glu Lys Lys Gly Glu Lys
   115 120 125
   Lys Glu Lys Lys Gln Gln Ser Ile Ala Gly Ser Ala Asp Ser Lys Pro
   130 135 140
   Ile Asp Val Ser Arg Leu Asp Leu Arg Ile Gly Cys Ile Ile Thr Ala
   145 150 155 160
   Arg Lys His Pro Asp Ala Asp Ser Leu Tyr Val Glu Glu Val Asp Val
   165 170 175
   Gly Glu Ile Ala Pro Arg Thr Val Val Ser Gly
   180 185
<210> 5
   <211> 192
   <212> PRT
   <213> Homo sapiens
<220>
   <221> PEPTIDE
   <222> (1)..(192)
   <223> AIMP1-(1-192)
<400> 5
   Met Ala Asn Asn Asp Ala Val Leu Lys Arg Leu Glu Gln Lys Gly Ala
   1 5 10 15
   Glu Ala Asp Gln Ile Ile Glu Tyr Leu Lys Gln Gln Val Ser Leu Leu
   20 25 30
   Lys Glu Lys Ala Ile Leu Gln Ala Thr Leu Arg Glu Glu Lys Lys Leu
   35 40 45
   Arg Val Glu Asn Ala Lys Leu Lys Lys Glu Ile Glu Glu Leu Lys Gln
   50 55 60
   Glu Leu Ile Gln Ala Glu Ile Gln Asn Gly Val Lys Gln Ile Ala Phe
   65 70 75 80
   Pro Ser Gly Thr Pro Leu His Ala Asn Ser Met Val Ser Glu Asn Val
   85 90 95
   Ile Gln Ser Thr Ala Val Thr Thr Val Ser Ser Gly Thr Lys Glu Gln
   100 105 110
   Ile Lys Gly Gly Thr Gly Asp Glu Lys Lys Ala Lys Glu Lys Ile Glu
   115 120 125
   Lys Lys Gly Glu Lys Lys Glu Lys Lys Gln Gln Ser Ile Ala Gly Ser
   130 135 140
   Ala Asp Ser Lys Pro Ile Asp Val Ser Arg Leu Asp Leu Arg Ile Gly
   145 150 155 160
   Cys Ile Ile Thr Ala Arg Lys His Pro Asp Ala Asp Ser Leu Tyr Val
   165 170 175
   Glu Glu Val Asp Val Gly Glu Ile Ala Pro Arg Thr Val Val Ser Gly
   180 185 190
<210> 6
   <211> 21
   <212> PRT
   <213> Homo sapiens
<220>
   <221> PEPTIDE
   <222> (1)..(21)
   <223> AIMP1-(14-34)
<400> 6
   Lys Gly Ala Glu Ala Asp Gln Ile Ile Glu Tyr Leu Lys Gln Gln Val
   1 5 10 15
   Ser Leu Leu Lys Glu
   20
<210> 7
   <211> 21
   <212> PRT
   <213> Homo sapiens
<220>
   <221> PEPTIDE
   <222> (1)..(21)
   <223> AIMP1-(26-46)
<400> 7
   Lys Gln Gln Val Ser Leu Leu Lys Glu Lys Ala Ile Leu Gln Ala Thr
   1 5 10 15
   Leu Arg Glu Glu Lys
   20
<210> 8
   <211> 312
   <212> PRT
   <213> Homo sapiens
<400> 8
   Met Ala Asn Asn Asp Ala Val Leu Lys Arg Leu Glu Gln Lys Gly Ala
   1 5 10 15
   Glu Ala Asp Gln Ile Ile Glu Tyr Leu Lys Gln Gln Val Ser Leu Leu
   20 25 30
   Lys Glu Lys Ala Ile Leu Gln Ala Thr Leu Arg Glu Glu Lys Lys Leu
   35 40 45
   Arg Val Glu Asn Ala Lys Leu Lys Lys Glu Ile Glu Glu Leu Lys Gln
   50 55 60
   Glu Leu Ile Gln Ala Glu Ile Gln Asn Gly Val Lys Gln Ile Ala Phe
   65 70 75 80
   Pro Ser Gly Thr Pro Leu His Ala Asn Ser Met Val Ser Glu Asn Val
   85 90 95
   Ile Gln Ser Thr Ala Val Thr Thr Val Ser Ser Gly Thr Lys Glu Gln
   100 105 110
   Ile Lys Gly Gly Thr Gly Asp Glu Lys Lys Ala Lys Glu Lys Ile Glu
   115 120 125
   Lys Lys Gly Glu Lys Lys Glu Lys Lys Gln Gln Ser Ile Ala Gly Ser
   130 135 140
   Ala Asp Ser Lys Pro Ile Asp Val Ser Arg Leu Asp Leu Arg Ile Gly
   145 150 155 160
   Cys Ile Ile Thr Ala Arg Lys His Pro Asp Ala Asp Ser Leu Tyr Val
   165 170 175
   Glu Glu Val Asp Val Gly Glu Ile Ala Pro Arg Thr Val Val Ser Gly
   180 185 190
   Leu Val Asn His Val Pro Leu Glu Gln Met Gln Asn Arg Met Val Ile
   195 200 205
   Leu Leu Cys Asn Leu Lys Pro Ala Lys Met Arg Gly Val Leu Ser Gln
   210 215 220
   Ala Met Val Met Cys Ala Ser Ser Pro Glu Lys Ile Glu Ile Leu Ala
   225 230 235 240
   Pro Pro Asn Gly Ser Val Pro Gly Asp Arg Ile Thr Phe Asp Ala Phe
   245 250 255
   Pro Gly Glu Pro Asp Lys Glu Leu Asn Pro Lys Lys Lys Ile Trp Glu
   260 265 270
   Gln Ile Gln Pro Asp Leu His Thr Asn Asp Glu Cys Val Ala Thr Tyr
   275 280 285
   Lys Gly Val Pro Phe Glu Val Lys Gly Lys Gly Val Cys Arg Ala Gln
   290 295 300
   Thr Met Ser Asn Ser Gly Ile Lys
   305 310
<210> 9
   <211> 936
   <212> DNA
   <213> Homo sapiens
<400> 9
   atggcaaata atgatgctgt tctgaagaga ctggagcaga agggtgcaga ggcagatcaa 60
   atcattgaat atcttaagca gcaagtttct ctacttaagg agaaagcaat tttgcaggca 120
   actttgaggg aagagaagaa acttcgagtt gaaaatgcta aactgaagaa agaaattgaa 180
   gaactgaaac aagagctaat tcaggcagaa attcaaaatg gagtgaagca aataccattt 240
   ccatctggta ctccactgca cgctaattct atggtttctg aaaatgtgat acagtctaca 300
   gcagtaacaa ccgtatcttc tggtaccaaa gaacagataa aaggaggaac aggagacgaa 360
   aagaaagcga aagagaaaat tgaaaagaaa ggagagaaga aggagaaaaa acagcaatca 420
   atagctggaa gtgccgactc taagccaata gatgtttccc gtctggatct tcgaattggt 480
   tgcatcataa ctgctagaaa acaccctgat gcagattctt tgtatgtgga agaagtagat 540
   gtcggagaaa tagccccaag gacagttgtc agtggcctgg tgaatcatgt tcctcttgaa 600
   cagatgcaaa atcggatggt gattttactt tgtaacctga aacctgcaaa gatgagggga 660
   gtattatctc aagcaatggt catgtgtgct agttcaccag agaaaattga aatcttggct 720
   cctccaaatg ggtctgttcc tggagacaga attacttttg atgctttccc aggagagcct 780
   gacaaggagc tgaatcctaa gaagaagatt tgggagcaga tccagcctga tcttcacact 840
   aatgatgagt gtgtggctac atacaaagga gttccctttg aggtgaaagg gaagggagta 900
   tgtagggctc aaaccatgag caacagtgga atcaaa 936
<210> 10
   <211> 35
   <212> DNA
   <213> Artificial Sequence
<220>
   <223> AIMP1-(1-192) sense primer
<400> 10
   cggaattcat ggcaaataat gatgctgttc tgaag 35
<210> 11
   <211> 32
   <212> DNA
   <213> Artificial Sequence
<220>
   <223> AIMP1-(1-192) antisense primer
<400> 11
   gtctcgagtt agccactgac aactgtcctt gg 32
<210> 12
   <211> 32
   <212> DNA
   <213> Artificial Sequence
<220>
   <223> AIMP1-(6-192) sense primer
<400> 12
   cggaattcgc tgttctgaag agactggagc ag 32
<210> 13
   <211> 32
   <212> DNA
   <213> Artificial Sequence
<220>
   <223> AIMP1-(6-192) antisense primer
<400> 13
   gtctcgagtt agccactgac aactgtcctt gg 32
<210> 14
   <211> 35
   <212> DNA
   <213> Artificial Sequence
<220>
   <223> AIMP1-(30-192) sense primer
<400> 14
   cggaattctc tctacttaag gagaaagcaa ttttg 35
<210> 15
   <211> 32
   <212> DNA
   <213> Artificial Sequence
<220>
   <223> AIMP1-(30-192) antisens primer
<400> 15
   gtctcgagtt agccactgac aactgtcctt gg 32
<210> 16
   <211> 35
   <212> DNA
   <213> Artificial Sequence
<220>
   <223> AIMP1-(47-192) sense primer
<400> 16
   cggaattcaa acttcgagtt gaaaatgcta aactg 35
<210> 17
   <211> 32
   <212> DNA
   <213> Artificial Sequence
<220>
   <223> AIMP1-(47-192) antisense primer
<400> 17
   gtctcgagtt agccactgac aactgtcctt gg 32
<210> 18
   <211> 35
   <212> DNA
   <213> Artificial Sequence
<220>
   <223> AIMP1-(54-192) sense primer
<400> 18
   cggaattcaa actgaagaaa gaaattgaag aactg 35
<210> 19
   <211> 32
   <212> DNA
   <213> Artificial Sequence
<220>
   <223> AIMP1-(54-192) antisens primer
<400> 19
   gtctcgagtt agccactgac aactgtcctt gg 32
<210> 20
   <211> 31
   <212> DNA
   <213> Artificial Sequence
<220>
   <223> AIMP1-(101-192) sense primer
<400> 20
   cggaattcgc agtaacaacc gtatcttctg 31
<210> 21
   <211> 32
   <212> DNA
   <213> Artificial Sequence
<220>
   <223> AIMP1-(101-192) antisense primer
<400> 21
   gtctcgagtt agccactgac aactgtcctt gg 32
<210> 22
   <211> 32
   <212> DNA
   <213> Artificial Sequence
<220>
   <223> AIMP1-(114-192) sense primer
<400> 22
   cggaattcaa aggaggaaca ggagacgaaa ag 32
<210> 23
   <211> 32
   <212> DNA
   <213> Artificial Sequence
<220>
   <223> AIMP-(114-192) antisense primer
<400> 23
   gtctcgagtt agccactgac aactgtcctt gg 32
<210> 24
   <211> 35
   <212> DNA
   <213> Artificial Sequence
<220>
   <223> AIMP1-(1-46) sense primer
<400> 24
   cggaattcat ggcaaataat gatgctgttc tgaag 35
<210> 25
   <211> 33
   <212> DNA
   <213> Artificial Sequence
<220>
   <223> AIMP1-(1-46) antisense primer
<400> 25
   gtctcgagtt acttctcttc cctcaaagtt gcc 33
<210> 26
   <211> 35
   <212> DNA
   <213> Artificial Sequence
<220>
   <223> AIMP-1-(1-53) sense primer
<400> 26
   cggaattcat ggcaaataat gatgctgttc tgaag 35
<210> 27
   <211> 33
   <212> DNA
   <213> Artificial Sequence
<220>
   <223> AIMP1-(1-53) antisense primer
<400> 27
   gtctcgagtt aagcattttc aactcgaagt ttc 33
<210> 28
   <211> 33
   <212> DNA
   <213> Artificial Sequence
<220>
   <223> AIMP1-(193-312) sense primer
<400> 28
   cggaattcct ggtgaatcat gttcctcttg aac 33
<210> 29
   <211> 33
   <212> DNA
   <213> Artificial Sequence
<220>
   <223> AIMP1-(193-312) antisense primer
<400> 29
   gtctcgagtt atttgattcc actgttgctc atg 33
<210> 30
   <211> 32
   <212> DNA
   <213> Artificial Sequence
<220>
   <223> AIMP1-(6-46) sense primer
<400> 30
   cggaattcgc tgttctgaag agactggagc ag 32
<210> 31
   <211> 35
   <212> DNA
   <213> Artificial Sequence
<220>
   <223> AIMP1-(6-46) antisense primer
<400> 31
   gtctcgagtt acttctcttc cctcaaagtt gcctg 35
<210> 32
   <211> 187
   <212> PRT
   <213> Homo sapiens
<400> 32
   Ala Val Leu Lys Arg Leu Glu Gln Lys Gly Ala Glu Ala Asp Gln Ile
   1 5 10 15
   Ile Glu Tyr Leu Lys Gln Gln Val Ser Leu Leu Lys Glu Lys Ala Ile
   20 25 30
   Leu Gln Ala Thr Leu Arg Glu Glu Lys Lys Leu Arg Val Glu Asn Ala
   35 40 45
   Lys Leu Lys Lys Glu Ile Glu Glu Leu Lys Gln Glu Leu Ile Gln Ala
   50 55 60
   Glu Ile Gln Asn Gly Val Lys Gln Ile Pro Phe Pro Ser Gly Thr Pro
   65 70 75 80
   Leu His Ala Asn Ser Met Val Ser Glu Asn Val Ile Gln Ser Thr Ala
   85 90 95
   Val Thr Thr Val Ser Ser Gly Thr Lys Glu Gln Ile Lys Gly Gly Thr
   100 105 110
   Gly Asp Glu Lys Lys Ala Lys Glu Lys Ile Glu Lys Lys Gly Glu Lys
   115 120 125
   Lys Glu Lys Lys Gln Gln Ser Ile Ala Gly Ser Ala Asp Ser Lys Pro
   130 135 140
   Ile Asp Val Ser Arg Leu Asp Leu Arg Ile Gly Cys Ile Ile Thr Ala
   145 150 155 160
   Arg Lys His Pro Asp Ala Asp Ser Leu Tyr Val Glu Glu Val Asp Val
   165 170 175
   Gly Glu Ile Ala Pro Arg Thr Val Val Ser Gly
   180 185
<210> 33
   <211> 187
   <212> PRT
   <213> Homo sapiens
<400> 33
   Ala Val Leu Lys Arg Leu Glu Gln Lys Gly Ala Glu Ala Asp Gln Ile
   1 5 10 15
   Ile Glu Tyr Leu Lys Gln Gln Val Ser Leu Leu Lys Glu Lys Ala Ile
   20 25 30
   Leu Gln Ala Thr Leu Arg Glu Glu Lys Lys Leu Arg Val Glu Asn Ala
   35 40 45
   Lys Leu Lys Lys Glu Ile Glu Glu Leu Lys Gln Glu Leu Ile Gln Ala
   50 55 60
   Glu Ile Gln Asn Gly Val Lys Gln Ile Ala Phe Pro Ser Gly Thr Pro
   65 70 75 80
   Leu His Ala Asn Ser Met Val Ser Glu Asn Val Ile Gln Ser Thr Ala
   85 90 95
   Val Thr Ala Val Ser Ser Gly Thr Lys Glu Gln Ile Lys Gly Gly Thr
   100 105 110
   Gly Asp Glu Lys Lys Ala Lys Glu Lys Ile Glu Lys Lys Gly Glu Lys
   115 120 125
   Lys Glu Lys Lys Gln Gln Ser Ile Ala Gly Ser Ala Asp Ser Lys Pro
   130 135 140
   Ile Asp Val Ser Arg Leu Asp Leu Arg Ile Gly Cys Ile Ile Thr Ala
   145 150 155 160
   Arg Lys His Pro Asp Ala Asp Ser Leu Tyr Val Glu Glu Val Asp Val
   165 170 175
   Gly Glu Ile Ala Pro Arg Thr Val Val Ser Gly
   180 185
<210> 34
   <211> 187
   <212> PRT
   <213> Homo sapiens
<400> 34
   Ala Val Leu Lys Arg Leu Glu Gln Lys Gly Ala Glu Ala Asp Gln Ile
   1 5 10 15
   Ile Glu Tyr Leu Lys Gln Gln Val Ser Leu Leu Lys Glu Lys Ala Ile
   20 25 30
   Leu Gln Ala Thr Leu Arg Glu Glu Lys Lys Leu Arg Val Glu Asn Ala
   35 40 45
   Lys Leu Lys Lys Glu Ile Glu Glu Leu Lys Gln Glu Leu Ile Gln Ala
   50 55 60
   Glu Ile Gln Asn Gly Val Lys Gln Ile Ala Phe Pro Ser Gly Thr Pro
   65 70 75 80
   Leu His Ala Asn Ser Met Val Ser Glu Asn Val Ile Gln Ser Thr Ala
   85 90 95
   Val Thr Thr Val Ser Ser Gly Thr Lys Glu Gln Ile Lys Gly Gly Ala
   100 105 110
   Gly Asp Glu Lys Lys Ala Lys Glu Lys Ile Glu Lys Lys Gly Glu Lys
   115 120 125
   Lys Glu Lys Lys Gln Gln Ser Ile Ala Gly Ser Ala Asp Ser Lys Pro
   130 135 140
   Ile Asp Val Ser Arg Leu Asp Leu Arg Ile Gly Cys Ile Ile Thr Ala
   145 150 155 160
   Arg Lys His Pro Asp Ala Asp Ser Leu Tyr Val Glu Glu Val Asp Val
   165 170 175
   Gly Glu Ile Ala Pro Arg Thr Val Val Ser Gly
   180 185
<210> 35
   <211> 192
   <212> PRT
   <213> Homo sapiens
<400> 35
   Met Ala Asn Asn Asp Ala Val Leu Lys Arg Leu Glu Gln Lys Gly Ala
   1 5 10 15
   Glu Ala Asp Gln Ile Ile Glu Tyr Leu Lys Gln Gln Val Ser Leu Leu
   20 25 30
   Lys Glu Lys Ala Ile Leu Gln Ala Thr Leu Arg Glu Glu Lys Lys Leu
   35 40 45
   Arg Val Glu Asn Ala Lys Leu Lys Lys Glu Ile Glu Glu Leu Lys Gln
   50 55 60
   Glu Leu Ile Gln Ala Glu Ile Gln Asn Gly Val Lys Gln Ile Pro Phe
   65 70 75 80
   Pro Ser Gly Thr Pro Leu His Ala Asn Ser Met Val Ser Glu Asn Val
   85 90 95
   Ile Gln Ser Thr Ala Val Thr Thr Val Ser Ser Gly Thr Lys Glu Gln
   100 105 110
   Ile Lys Gly Gly Thr Gly Asp Glu Lys Lys Ala Lys Glu Lys Ile Glu
   115 120 125
   Lys Lys Gly Glu Lys Lys Glu Lys Lys Gln Gln Ser Ile Ala Gly Ser
   130 135 140
   Ala Asp Ser Lys Pro Ile Asp Val Ser Arg Leu Asp Leu Arg Ile Gly
   145 150 155 160
   Cys Ile Ile Thr Ala Arg Lys His Pro Asp Ala Asp Ser Leu Tyr Val
   165 170 175
   Glu Glu Val Asp Val Gly Glu Ile Ala Pro Arg Thr Val Val Ser Gly
   180 185 190
<210> 36
   <211> 192
   <212> PRT
   <213> Homo sapiens
<400> 36
   Met Ala Asn Asn Asp Ala Val Leu Lys Arg Leu Glu Gln Lys Gly Ala
   1 5 10 15
   Glu Ala Asp Gln Ile Ile Glu Tyr Leu Lys Gln Gln Val Ser Leu Leu
   20 25 30
   Lys Glu Lys Ala Ile Leu Gln Ala Thr Leu Arg Glu Glu Lys Lys Leu
   35 40 45
   Arg Val Glu Asn Ala Lys Leu Lys Lys Glu Ile Glu Glu Leu Lys Gln
   50 55 60
   Glu Leu Ile Gln Ala Glu Ile Gln Asn Gly Val Lys Gln Ile Ala Phe
   65 70 75 80
   Pro Ser Gly Thr Pro Leu His Ala Asn Ser Met Val Ser Glu Asn Val
   85 90 95
   Ile Gln Ser Thr Ala Val Thr Ala Val Ser Ser Gly Thr Lys Glu Gln
   100 105 110
   Ile Lys Gly Gly Thr Gly Asp Glu Lys Lys Ala Lys Glu Lys Ile Glu
   115 120 125
   Lys Lys Gly Glu Lys Lys Glu Lys Lys Gln Gln Ser Ile Ala Gly Ser
   130 135 140
   Ala Asp Ser Lys Pro Ile Asp Val Ser Arg Leu Asp Leu Arg Ile Gly
   145 150 155 160
   Cys Ile Ile Thr Ala Arg Lys His Pro Asp Ala Asp Ser Leu Tyr Val
   165 170 175
   Glu Glu Val Asp Val Gly Glu Ile Ala Pro Arg Thr Val Val Ser Gly
   180 185 190
<210> 37
   <211> 192
   <212> PRT
   <213> Homo sapiens
<400> 37
   Met Ala Asn Asn Asp Ala Val Leu Lys Arg Leu Glu Gln Lys Gly Ala
   1 5 10 15
   Glu Ala Asp Gln Ile Ile Glu Tyr Leu Lys Gln Gln Val Ser Leu Leu
   20 25 30
   Lys Glu Lys Ala Ile Leu Gln Ala Thr Leu Arg Glu Glu Lys Lys Leu
   35 40 45
   Arg Val Glu Asn Ala Lys Leu Lys Lys Glu Ile Glu Glu Leu Lys Gln
                   50                  55                  60
   Glu Leu Ile Gln Ala Glu Ile Gln Asn Gly Val Lys Gln Ile Ala Phe
   65                  70                  75                  80
   Pro Ser Gly Thr Pro Leu His Ala Asn Ser Met Val Ser Glu Asn Val
                   85                  90                  95
   Ile Gln Ser Thr Ala Val Thr Thr Val Ser Ser Gly Thr Lys Glu Gln
               100                 105                 110
   Ile Lys Gly Gly Ala Gly Asp Glu Lys Lys Ala Lys Glu Lys Ile Glu
           115                 120                 125
   Lys Lys Gly Glu Lys Lys Glu Lys Lys Gln Gln Ser Ile Ala Gly Ser
       130                 135                 140
   Ala Asp Ser Lys Pro Ile Asp Val Ser Arg Leu Asp Leu Arg Ile Gly
   145                 150                 155                 160
   Cys Ile Ile Thr Ala Arg Lys His Pro Asp Ala Asp Ser Leu Tyr Val
                   165                 170                 175
   Glu Glu Val Asp Val Gly Glu Ile Ala Pro Arg Thr Val Val Ser Gly
               180                 185                 190

## Description

### Technical Field

The present invention relates to a novel peptide and the use thereof. More particularly, the present invention relates to a peptide having the activity of promoting wound healing by stimulating the proliferation of fibroblast cells.

### Background Art

Wound healing refers to the repair or replacement of injured tissues, including the skin, muscle, nervous tissue, bone, soft tissue, internal organ and blood vessel tissue. Such wound healing results from a series of tissue responses, such as acute and chronic inflammation, cellular migration, angiogenesis and extracellular matrix (ECM) accumulation. If a wound occurs, it will cause damage to the blood vessels of the surrounding tissue to cause bleeding in lesions. When fibrinogen in the blood clot forms fibrin gel, plasma proteins, such as fibronectin will invade the gel. In addition, inflammatory cells, fibroblast cells, new blood vessel-forming cells and the like will invade this gel to accumulate ECM components, such as collagen and proteoglycan, in the tissue around the wound. For this reason, the originally existing fibrin matrix will be replaced by granulation tissue, and a scar will be formed at that site with the passage of time. Also, at the same time as the accumulation of the ECM components, keratinocytes will migrate to form an epithelial membrane that prevents the loss of water and the invasion of bacteria. A series of these processes associated with wound healing occur by the interaction between cells in injured tissue, such as immune cells, inflammatory cells and mesenchymal cells, various cytokines, such as transforming growth factor-β (TGF-β), platelet-derived growth factor (PDGF), epidermal growth factor (EGF), fibroblast growth factor (FGF) and fibroblast activation factor (FAF), and ECMs, such as collagen, fibronectin, tenascin and proteoglycan.

As drugs for promoting wound healing, compositions comprising cytokines associated with the above-described healing have been recently developed. For example, a wound healing agent of Becaplermin, a genetically manufactured PDGF, is commercially available from Jonshon & Jonhson, and a pharmaceutical composition for the regeneration and repair of mammalian tissues, comprising PDGF and dexamethasone, is disclosed in European Patent 0575484. Also, US Patent No. 5981606 discloses a wound-healing agent comprising TGF-β.

US2005/119175 and Park Sang Gyu et al: 'The novel cytokine p43 stimulates dermal fibroblast proliferation and wound repair'. The American Journal of Pathoolgy February 2005 volume 166, no. 2, February 2005, pages 387-398 disclose full-length P43 and its use.

WO2006/083087 discloses AIMP1 and it use.

Particularly, FGF among the cytokines associated with wound healing is known to promote wound healing by stimulating the proliferation of fibroblast cells, and there are wound-treating agents developed using FGF. Namely, US Patent No. 6,800,286 discloses a chimeric FGF having the activity of promoting wound healing, and US Patent No. 5,155,214 discloses a wound-healing agent comprising FGF.

Meanwhile, a peptide consisting of numerous amino acids has shortcomings in that it is metabolized upon in vivo administration, leading to the cleavage of the peptide bond, and tends to decompose in a process of formulation. Thus, it is generally preferable to keep the length of peptides as short as possible for use as drugs. However, because the pharmacological activity of peptides needs to be kept, it is important in the development of drugs to find the minimum length peptide(s) with activity comparable to that of a long-chain peptide.

### Disclosure of the Invention

Accordingly, the present inventors have made many studies to develop a novel wound-healing agent, and as a result, found that a peptide comprising a portion of the amino acid sequence of the N-terminal region of the known AIMPI had the activity of promoting wound healing by stimulating the proliferation of fibroblast cells, thereby completing the present invention, which is defined by the claims.

Therefore, it is an object of the present invention to provide an isolated peptide consisting of a peptide in the range of 21-41 contiguous amino acids selected from the amino acid sequence of SEQ ID NO: 1 or the amino acid sequences having at least 90% sequence identity to the amino acid sequence of SEQ ID NO:1 and use thereof.

To achieve the above object, in one aspect, the present invention provides an isolated peptide consisting of a peptide in the range of 21-41 contiguous amino acids selected from the amino acid sequence of SEQ ID NO: 1 or the amino acid sequence having at least 90% sequence homology to the amino acid sequence of SEQ ID NO:1.

In another aspect, the present invention provides a composition comprising the peptide.

In still another aspect, the present invention provides the peptides of the invention for use in a method for promoting wound healing, which comprises administering to a subject in need thereof an effective amount of the peptide.

Hereinafter, the present invention will be described in detail.

### Definition

Unless otherwise stated, all technical and scientific terms used herein have the same meanings as commonly understood by those skilled in the art to which the present invention pertains.

As used herein, the term "wound" refers to the injured condition of a living body and encompasses pathological conditions under which tissues constituting the internal and external surface of the living body, for example, the skin, muscle, nervous tissue, bone, soft tissue, internal organs and vascular tissue, have been disrupted or broken. Examples of wounds include, but are not limited to, contusion or bruise, non-healing traumatic wounds, tissue disruption caused by irradiation, abrasion, gangrene, laceration, avulsion, penetrated wound, gun shot wound, cutting, burn, frostbite, skin ulcer, xeroderma, skin keratosis, breakage, rupture, dermatitis, smart caused by dermatophyte, surgical wound, wound caused by vascular disorders, corneal wound, sores such as pressure sores and bed sores, diabetes and poor circulation-associated conditions such as diabetic skin erosion, chronic ulcers, suture site following plastic surgery, spinal traumatic wound, gynecological wound, chemical wound and acne. Any damaged or injured part of a subject is within the definition of the wounds.

As used herein, the term "promoting wound healing" refers to repairing, replacing, alleviating, accelerating or curing the injured tissue of a subject.

As used herein, the term "effective amount" refers to an amount effective in stimulating the proliferation of fibroblast cells or promoting wound healing *in vivo* or *in vitro.*

As used herein, the term "subject" is intended to include mammals, and particularly animals including human beings, or the skin cells or skin tissues of animals. The subject may be a patient in need of treatment. Also, the skin cells may preferably be fibroblast cells.

A peptide according to the present invention comprises a portion of the N-terminal amino acid sequence of an Aminoacyl tRNA synthetases (ARS)-interacting multi-functional protein 1 (AIMP1). Meanwhile, the AIMP1 is previously known as the p43 protein and renamed by the present inventors (Sang Gyu Park, et al., Trends in Biochemical Sciences, 30:569-574, 2005).

The AIMP1 is a protein consisting of 312 amino acids, which binds to a multi-ARS complex to increase the catalytic activity of the multi-ARS complex. It is known that the AIMP1 is secreted from various types of cells, including prostate cancer cells, immune cells and transgenic cells, and the secreted AIMP1 works on diverse target cells such as monocytes, macrophages, endothelial cells and fibroblast cells. Three SNPs for the AIMP1 are known (see NCBI SNP database). Namely, the following SNPs are known: substitution of 79^{th} alamine (Ala) to proline (Pro) (SNP accession no. rs3133166); substitution of 104^{th} threonine (Thr) to alanine (Ala) (SNP accession no. rs17036670); and substitution of 117^{th} threonine (Thr) to alanine (Ala) (SNP accession no. rs2230255) in the amino acid sequence of the full-length AIMP1 (SEQ ID NO: 8).

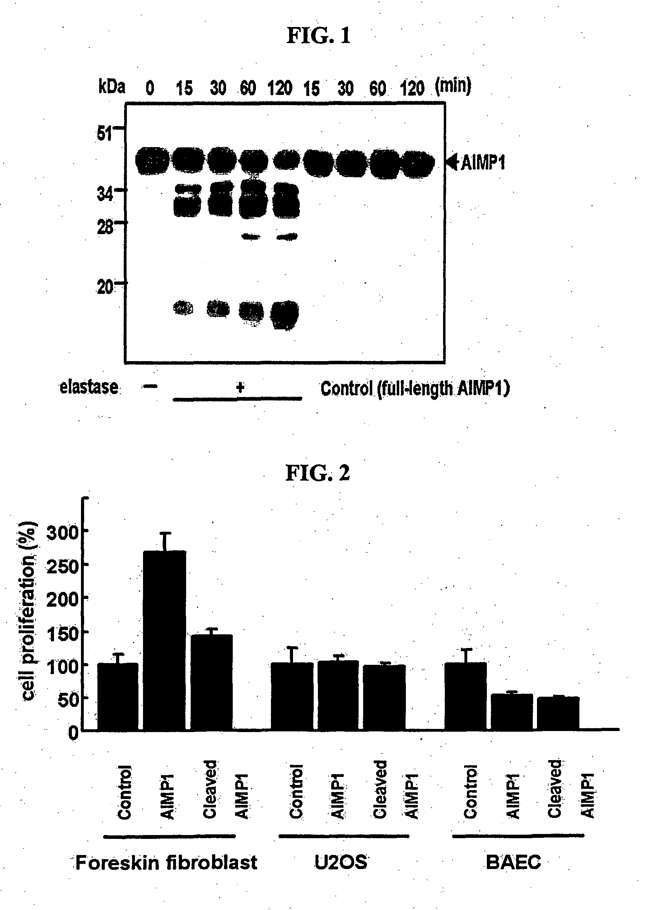

Meanwhile, the present inventors hypothesized that, because the AIMS1 has various complex activities in various different target cells, the AIMP1 is likely to use different structural motifs or domains for its diverse activities. To confirm this possibility, the present inventors cleaved the AIMP1 with proteases and examined whether the cleaved fragments of the AIMP1 still have activities (see Example 1). As a result, it was found that, when the AIMP1 was cleaved with elastase 2, it would be separated into small fragments (see FIG. 1), and the elastase 2-cleaved fragments would maintain pro-apoptotic activity (cell proliferation inhibitory activity) on endothelial cell, but lost growth-stimulating activity in fibroblast cells (see FIG. 2).

Thus, in order to determine the functional domain of the AIMP1, associated with the stimulation of fibroblast proliferation, the present inventors identified the elastase 2-cleavage sites of the AIMP1 (see FIG. 3), and constructed a series of deletion fragments of AIMP1 on the basis of the digestion results (see FIGS. 4 and 5) and then examined the activity of each of the fragments in the proliferation of fibroblast cells (see Example 2). As a result, it could be supposed that a region of amino acid 6-46 of the AIMP1 would be a domain having the activity of stimulating the proliferation of fibroblast cells (see FIG. 6).

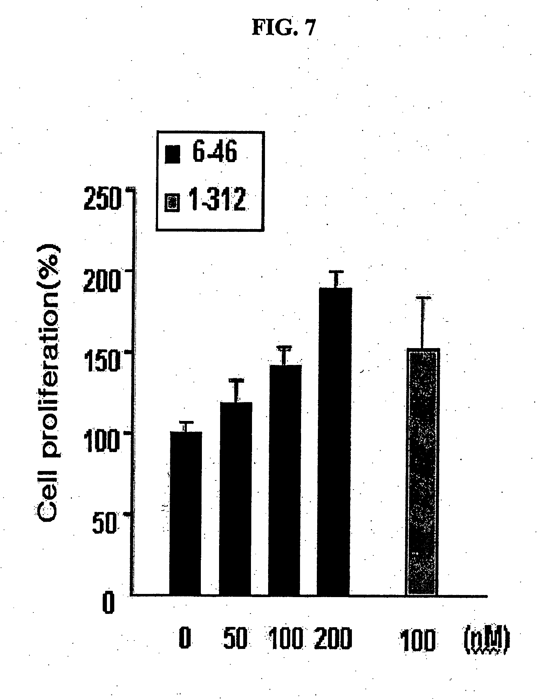

To prove this hypothesis, the present inventors synthesized a peptide corresponding to a region of amino acids 6-46 of the AINIP1 (see Example <3-1>), and measured the activity of the prepared peptide in the stimulation of fibroblast proliferation (see Example <3-2>). As a result, it could be found that the region of amino acid 6-46 of the AIMP1 stimulated the proliferation of fibroblast cells in a dose-dependent manner (see FIG. 7).

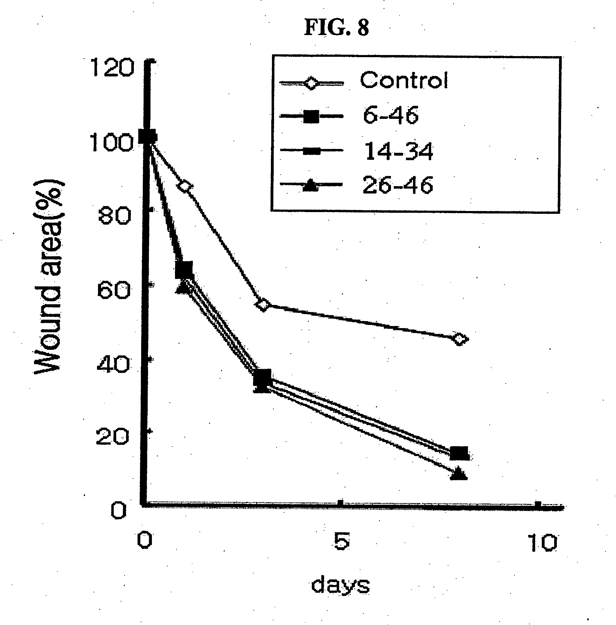

Furthermore, the present inventors examined whether the region of amino acid 6-46 of the AIMP1 could promote wound healing by stimulating the proliferation of fibroblast cells using an *in vivo* model. For this purpose, the region of amino acid 6-46 of the AIMP1 was randomly cleaved to prepare small fragments each consisting of 21 amino acids, and the activity of these fragments in the promotion of wound healing was also examined (see Example 4). As a result, it could be found that, not only the region of amino acids 6-46 of the AIMP1, but also the small fragments prepared therefrom, had the activity of promoting wound healing (see FIG. 8).

Accordingly, the present invention provides an isolated peptide consisting of either an amino acid sequence of SEQ ID NO: 1 or a peptide in the range of 21-41 contiguous amino acid sequence of SEQ ID NO: 1.
NH₂-AVLKRLEQKGAEADQIIEYLKQQVSLLKEKAILQATLREEK-COOH (SEQ ID NO: 1)

The definition of abbreviations used in the present invention is as follows: A (alanine); D (asparaginic acid); E (glutamic acid); G (glycine); I (isoleusine); K (lysine); L (leusine); Q (glutamine); R (arginine); S (serine); T (threonine); V (valine); and Y (tyrosine).

Preferably, the inventive peptide may have the amino acid sequence selected from the group consisting of SEQ ID NO: 1-3 or SEQ ID NO: 6-7. The amino acid sequence selected from SEQ ID NO: 32 to SEQ ID NO: 37 is a single nucleotide polymorphism (SNP) of SEQ ID NO: 4 or SEQ ID NO: 5. Most preferably, the inventive peptide may have the amino acid sequence of SEQ ID NO: 1.

Also, the inventive peptide may include functional equivalents of the peptide comprising 21-41 contiguous amino acids of the amino acid sequence of SEQ ID NO: 1, and preferably functional equivalents of the peptide having the amino acid sequence of SEQ ID NO: 1, as well as salts thereof. The term "functional equivalents" refer to peptides which have at least 90% amino acid sequence identity with the amino acid sequence set forth in SEQ ID NO: 1, preferably at least 95%, for example, 90%, 91%, 92%, 93%, 94%, 95%, 96%, 97%, 98%, 99% and 100% and exhibit substantially identical physiological activity to the polypeptide of SEQ ID NO: 1. The functional equivalents may include, for example peptides produced by as a result of addition, substitution or deletion of some amino acid of SEQ ID NO:1. Sequence identity is defined herein as the percentage of amino acid residues in the candidate sequence that are identical with amino acid sequence of SEQ ID NO: 1, after aligning the sequences and introducing gaps, if necessary, to achieve the maximum percent sequence identity, and not considering any conservative substitutions (as described above) as part of the sequence identity. None of N-terminal, C-terminal, or internal extensions, deletions, or insertions into the amino acid sequence of SEQ ID NO: 1 shall be construed as affecting sequence identity or homology. Thus, sequence identity can be determined by standard methods that are commonly used to compare the similarity in position of the amino acids of two polypeptides. Using a computer program such as BLAST or FASTA, two polypeptides are aligned for optimal matching of their respective amino acids (either along the full length of one or both sequences or along a predetermined portion of one or both sequences). The programs provide a default opening penalty and a default gap penalty, and a scoring matrix such as PAM 250 (a standard scoring matrix; see Dayhoff et al., in Atlas of Protein Sequence and Structure, vol. 5, supp. 3 (1978)) can be used in conjunction with the computer program. For example, the percent identity can be calculated as: the total number of identical matches multiplied by 100 and then divided by sum of the length of a longer sequence within the matched span and the number of gaps introduced into longer sequences in order to align the two sequences.

As used herein, the term "substantially identical physiological activity" refers to the activity that acts in fibroblast cells to stimulate the proliferation of fibroblast cells and to promote wound healing. The scope of the functional equivalents as used herein encompasses derivatives obtained by modifying a part of the chemical structure of the peptide set forth in SEQ ID NO: 1 while maintaining the basic framework and fibroblast-proliferating and wound healing-promoting activities of the peptide. For example, this includes structural modifications for altering the stability, storage, volatility or solubility of the peptide.

The peptide according to the present invention can be prepared by a genetic engineering method using the expression of recombinant nucleic acid encoding the same. For example, the inventive peptide can be prepared by a genetic engineering method comprising the steps of: inserting a nucleic acid sequence or its fragment encoding the inventive peptide into a recombinant vector comprising one or more expression control sequences which are operatively linked to the nucleic acid sequence to control the expression of the nucleic acid sequence; transforming a host cell with the resulting recombinant expression vector; culturing the transformed cell in a medium and condition suitable to express the nucleic acid sequence; and isolating and purifying a substantially pure protein from the culture medium. Genetic engineering methods for preparing peptides are known in the art (Maniatis et al., Molecula Cloning: A Laboratory Manual, Cold Spring Harbor Laboratory (1982); Sambrook et al., supra; Gene Expression Technology, Method in Enzymology, Genetics and Molecular Biology, Methods in Enzymology, Guthrie & Fink (eds.), Academic Press, San Diego, Calif. (1991); Hitzeman et al., J. Biol. Chem., 255, 12073-12080 (1980)).

Alternatively, the inventive peptide can be chemically synthesized according to any technique known in the art (Creighton, Proteins: Structures and Molecular Principles, W.H. Freeman and Co., NY 1983). Namely, the inventive peptide can be prepared by conventional liquid or solid phase synthesis, fragment condensation, F-MOC or T-BOC chemistry (Chemical Approaches to the Synthesis of Peptides and Proteins, Williams et al., Eds., CRC Press, Boca Raton Florida, 1997; A Practical Approach, Atherton & Sheppard, Eds., IRL Press, Oxford, England, 1989).

It is particularly preferred to use the solid phase synthesis to prepare the inventive peptide. The inventive peptide can be synthesized by performing the condensation reaction between protected amino acids by the conventional solid-phase method, beginning with the C-terminal and progressing sequentially with the first amino acid, the second amino acid, the third amino acid, and the like according to the identified sequence. After the condensation reaction, the protecting groups and the carrier connected with the C-terminal amino acid may be removed by a known method such as acid decomposition or aminolysis. The above-described peptide synthesis method is described in detail in the literature (Gross and Meienhofer's, The peptides, vol. 2, Academic Press, 1980).

Examples of a solid-phase carrier, which can be used in the synthesis of the peptide according to the present invention, include polystyrene resins of substituted benzyl type, polystyrene resins of hydroxymethylphenylacetic amide form, substituted benzhydrylpolystyrene resins and polyacrylamide resins, having a functional group capable of bonding to peptides. Also, the condensation of amino acids can be performed using conventional methods, for example dicyclohexylcarbodimide (DDC) method, acid anhydride method and activated ester method.

Protecting groups used in the synthesis of the inventive peptide are those commonly used in peptide syntheses, including those readily removable by conventional methods such as acid decomposition, reduction or aminolysis. Specific examples of such amino protecting groups include formyl; trifluoroacetyl; benzyloxycarbonyl; substituted benzyloxycarbonyl such as (ortho- or para-) chlorobenzyloxycarbonyl and (ortho- or para-) bromobenzyloxycarbonyl; and aliphatic oxycarbonyl such as t-butoxycarbonyl and t-amiloxycarbonyl. The carboxyl groups of amino acids can be protected through conversion into ester groups. The ester groups include benzyl esters, substituted benzyl esters such as methoxybenzyl ester; alkyl esters such as cyclohexyl ester, cycloheptyl ester or t-butyl ester. The guanidino moiety may be protected by nitro; or arylsulfonyl such as tosyl, methoxybenzensulfonyl or mesitylenesulfonyl, even though it does not need a protecting group. The protecting groups of imidazole include tosy, benzyl and dinitrophenyl. The indole group of tryptophan may be protected by formyl or may not be protected.

Deprotection and separation of protecting groups from carriers can be carried out using anhydrous hydrofluoride in the presence of various scavengers. Examples of the scavengers include those commonly used in peptide syntheses, such as anisole, (ortho-, meta- or para-) cresol, dimethylsulfide, thiocresol, ethanendiol and mercaptopyridine.

The recombinant peptide prepared by the genetic engineering method or the chemically synthesizing can be isolated and purified according to methods known in the art, including extraction, recrystallization, various chromatographic techniques (e.g., gel filtration, ion exchange, precipitation, adsorption, reverse phase, etc.), electrophoresis and counter current distribution.

The inventive peptide can be provided in the form of a composition. Also, the inventive composition may comprise a pharmaceutically acceptable salt of the inventive peptide as an active ingredient. Examples of the pharmaceutically acceptable salt include salts with inorganic bases, salts with organic bases, salts with inorganic acids, salts with organic acids, salts with basic or acidic amino acids and the like. Examples of the salt with an inorganic acid include alkali metal salts, such as a sodium salt and a potassium salt; an alkaline earth metal salt such as a calcium salt and a magnesium salt; an aluminum salt; and an ammonium salt. Examples of the salt with an organic base include salts with trimethylamine, triethylamine, pyridine, picoline, 2,6-lutidine, ethanolamine, diethanolamine, triethanolamine, cyclohexylamine, dicyclohexylamine and N,N'-dibenzylethylenediamine. Examples of the salt with an inorganic acid include salts with hydrochloric acid, boric acid, nitric acid, sulfuric acid and phosphoric acid. Examples of the salt with an organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid and p-toluenesulfonic acid. Examples of the salt with a basic amino acid include salts with arginine, lysine and ornithine. Examples of the salt with an acidic amino acid include salts with aspartic acid and glutamic acid. The list of suitable salts is disclosed in Remington's Pharmaceutical Sciences, 17th ed., Mack Publishing Company, Easton, PA, p1418, 1985.

Meanwhile, the inventive composition may further comprise a pharmaceutically acceptable carrier and can be formulated in any form according to any method known in the art. Preferably, it can be formulated in the form of external preparations. The preparation forms of the inventive composition include, but are not limited to, liquid coatings, sprays, lotions, creams, gels, pastes, ointments, aerosols, powders and transdermal delivery agents.

A pharmaceutically acceptable carrier, which can be used in the external preparations, can be selected depending on the dosage form of the inventive composition, and examples thereof include, but are not limited to, hydrocarbons such as vaseline, liquid paraffin, and plasticized hydrocarbon gel (plastibase); animal and vegetable oils such as medium-chain fatty acid triglyceride, lard, hard fat, and cacao butter; high fatty acid alcohols, fatty acids and esters thereof, such as cetanol, stearyl alcohol, stearic acid and isopropyl palimitate; water-soluble bases, such as polyethylene glycol, 1,3-butylene glycol, glycerol, gelatin, white sugar, and sugar alcohol; emulsifiers such as glycerine fatty acid ester, polyoxyl stearate, and polyoxyethylene hydrogenated castor oil; thickeners such as acrylic acid esters and sodium alginates; propellants, such as liquefied petroleum gas and carbon dioxide; and preservatives, such as paraoxybenzoic acid esters. In addition to these carriers, additives such as stabilizers, pigments, coloring agents, pH adjusting agents, diluents, surfactants, preservatives and antioxidants may, if necessary, be contained in the inventive external preparation. The external preparation of the present invention can preferably be applied to a local wound site by conventional methods.

The external preparations may be used for adhesion to a conventional solid support such as the wound release cover of an adhesive bandage. The adhesion can be achieved by saturating the solid support with the inventive composition and then dehydrating the composition. In a preferred embodiment, the solid support may be coated with an adhesive agent to improve the adhesion of the inventive composition to the solid support. Examples of the adhesive agent include polyacrylate and cyanoacrylate. This type of preparations are commercially available, and examples thereof include bandages having a non-adhesive wound release cover in the form of perforated plastic film (Smith & Nephew Ltd.); thin strips, patches, spots and BAND-AID in the form of a thermoplastic strip, commercially available from Johnson & Johnson; CURITY CURAD (ouchless bandage) commercially available from Kendall Co. (a division of Colgate-Palmolive Co.); and STIK-TITE (elastic strip) commercially available from American White Cross Laboratories, Inc. The inventive peptide can be applied as an active ingredient in this type of preparations.

Furthermore, the inventive composition may also be formulated as preparations for oral administration. For oral administration, the inventive peptide can be formulated in the form of ingestible tablets, buccal tablets, troches, capsules, elixirs, suspensions, syrups, wafers, and the like. These preparations may also comprise diluents (e.g., lactose, dextrose, sucrose, mannitol, sorbitol, cellulose and/or glycine), lubricants (e.g., silica, talc, stearic acid and a magnesium or calcium salt thereof, and/or polyethylene glycol) in addition to the active ingredient. Among various preparations, tablets may also comprise binders, such as magnesium aluminum silicate, starch pastes, gelatin, tragacanth, methylcellulose, sodium carboxymethylcellulose and/or polyvinylpyrrolidone, and, if desired, may further comprise disintegrating agents, such as starches, agar or alginic acid or a sodium salt thereof, absorbents, colorants, flavors and sweeteners.

Also, the inventive composition may further comprise other active ingredient having an activity of promoting fibroblast proliferation and wound healing and known in the art to enhance the activity of inventive peptide. For example, the inventive composition may further comprise antibiotics, such as tetracycline, oxytetracycline, gentamicin, neomycin sulfate, bacitracin and polymyxin B sulfate; antihistamines, such as diphenhydramine, promethazine, tripelennamine, phenothialzine, chlorophenylamine, antazoline and pantholin; anti-inflammatory drugs; anti-viral drugs; anti-fungal agents; and growth factors, such as PDGF, PDAF, PDEGF, TGF- β, PF-4, α -FGF, bFGF, vascular endothelial growth factor (VEGF), growth hormone (GH), EGF and insulin-like growth factor (IGF).

In still another aspect, the present invention provides the peptides of the invention for use in a method for promoting wound healing, which comprises administering to a subject in need thereof an effective amount of the inventive peptide.

The inventive peptide may be administered itself or in the form of various formulations as described above, and preferably it may be administered until the desired effect, i.e., effect of promoting fibroblast proliferation and wound healing is achieved.

Also, the inventive peptide may be administered by various routes according to any method known in the art. Namely, it may be administered by oral or parenteral routes. For example, the parenteral routes include methods for applying to the skin locally, intramuscular, intravenous, intracutaneous, intraarterial, intramarrow, intrathecal, intraperitoneal, intranasal, intravaginal, intrarectal, sublingual and subcutaneous or administering to gastrointestinal tracts, mucosa or respiratory organs systemically. Preferably, the inventive peptide may be administered by a method of applying the polypeptide directly to the skin.

The effective amount of the inventive peptide may be suitably determined by considering various factors, such as age, body weight, health condition, sex, disease severity, diet and excretion of a subject in need of treatment, as well as administration time and administration route. Preferably, the effective amount of the inventive peptide is about 1 to 10000 µg/kg body weight/day, more preferably 10 to 1000 µg/kg body weight/day.

Hereinafter, the present invention will be described in detail by examples. It is to be understood, however, that these examples are for illustrative purpose only and are not construed to limit the scope of the present invention. Peptides not encompassed by the claims are for comparative purposes only.

### Brief Description of the Drawings

FIG. 1 shows the results of SDS-PAGE analysis for an AIMP1 treated with elastase 2.
   -: untreated with elastase 2; and
   +: treated with elastase 2.
FIG. 2 shows analysis results for cell proliferation activities of AIMP1 fragments cleaved by elastase 2 in various cells.

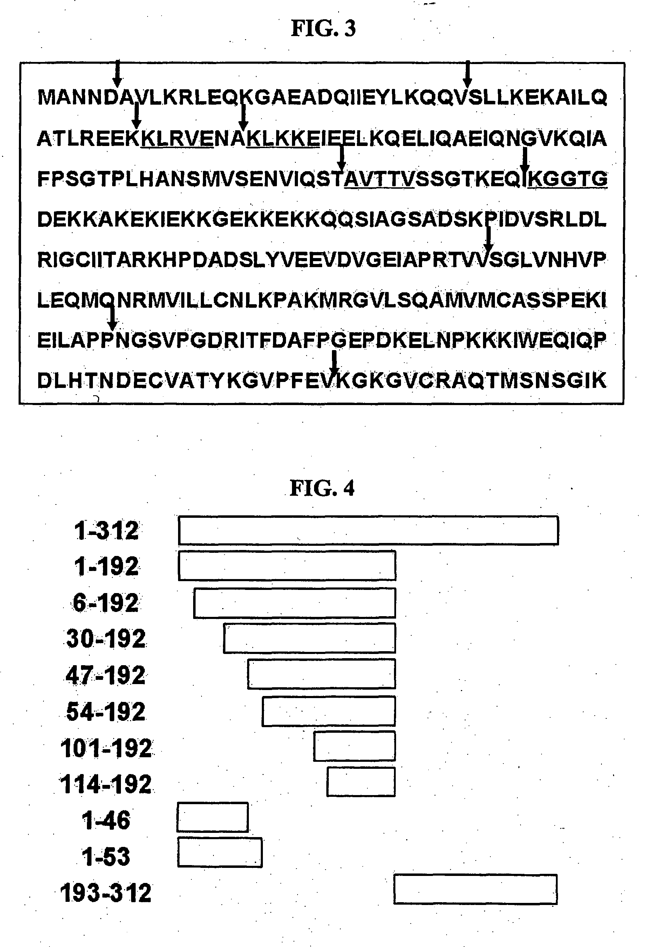

FIG. 3 shows the results by N-terminal amino acid sequencing of N-terminal amino acids of elastase 2-cleaved AIMP1 fragments and identification of cleavage sites.
   Arrows: digestion sites
FIG. 4 is a schematic diagram showing deletion mutants of AIMP1 according to the present invention.

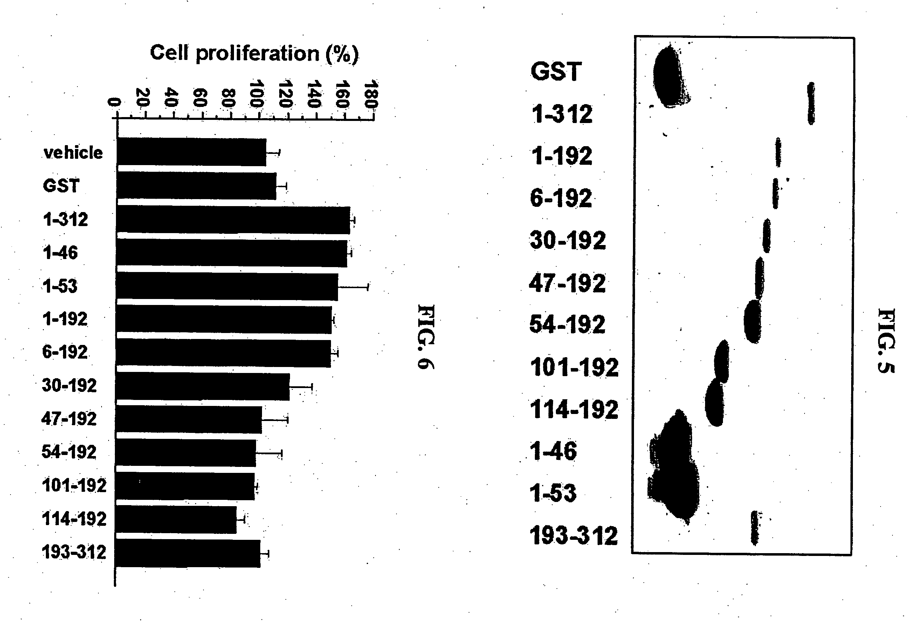

FIG. 5 shows the results of SDS-PAGE analysis for deletion mutants of recombinant AIMP1 according to the present invention.
FIG. 6 shows measurement results for the fibroblast proliferation-stimulating activities of deletion mutant fragments of AIMP1 according to the present invention.
FIG. 7 shows measurement results for the fibroblast proliferation-stimulating activity of an AIMP1-(6-46) fragment according to the present invention.
FIG. 8 shows *in vivo* measurement results for the wound healing-promoting activities of deletion mutant fragments of AIMP1 according to the present invention.

### Best Mode for Carrying Out the Invention

### Test Example 1: Measurement of activities of AIMP fragments cleaved by elastase 2

### <1₋1> Treatment with elastase 2

An AIMP1 (SEQ ID NO: 8) consisting of 312 amino acids was prepared according to the method of Park et al. (Park S. G. et al., J. Biol. Chem., 277:45243-45248, 2002). The AIMP1 was treated with elastase 2, and various cells were treated with the cleaved AIMP1 fragments to measure the cell proliferation activities of the fragments. Namely, 4 µg of the AIMP1 was cultured with 1 unit/ml of elastase 2 at 37 °C for 4 hours. After completion of the culture, the collected fragments were analyzed by 15% SDS-PAGE and visualized by staining with coomassie blue.

From the test results, it could be found that the AIMP1 was separated into small fragments by treatment with elastase 2 (see FIG. 1).

### <1-2> Cell proliferation activities of AIMP fragments cleaved by elastase 2

Human foreskin fibroblast cells (MC1232 obtained from MIT), U2OS cells (ATCC HTB-96) and bovine aorta endotheilial cells (hereinafter, referred to as "BAECs") were treated with the AIMP1 fragments cleaved by elastase 2 in Test Example <1-1>, and the effects of the AIMP1 fragments on the proliferation of the cells were examined. The U2OS cells, osteosarcomas, were used in a control group, because it is known that cell proliferation by AIMP1 does not occur in these cells.

Bovine aorta endothelial cells (BAECs) were isolated from bovine descending thoracic aorta and cultured in Dulbecco's modified Eagle's medium (DMEM) containing 20% FBS at 37 °C in a 5% CO₂ atmosphere. The foreskin fibroblast cells and the U2OS cells were cultured in DMEM with 10% FBS and 1% antibiotic. Each of the cultured cell lines (5x10³ cells) was cultivated in a 24-well dish for 12 hours and then subjected to serum-starved culture for 3 hours. Next, 100 nM of a full-length AIMP1 or fragments of AIMP1 cleaved with an enzyme were added to each well and cultivated for 12 hours. The control group was without the testing agents. After completion of the cultivation, tritium-labeled thymidine (1µCi/well) was added to each well, and the cells were additionally cultured at 37°C for 4 hours. The cultured cells were washed with PBS three times and fixed with 5% TCA for 10 minutes, followed by washing again with PBS three times. The cells were lysed with 0.5N NaOH, and the incorporated thymidine was quantified with a liquid scintillation counter.

The test results showed that the elastase 2-cleaved AIMP1 fragments maintained the activity of inhibiting the proliferation of endothelial cells in the same manner as the full-length AIMP1, but lost the activity of inducing the proliferation of fibroblast cells (see FIG. 2). From these results, it could be found that various activities of the AIMP1 appeared by different domains present in the AIMP1 and it could be supposed that the region of AIMP1 cleaved by elastase 2 performed an important role in the proliferation of fibroblast cells.

### Example 1: Identification of elastase 2-cleaved sites of AIMP1 and construction of AIMP1 deletion fragments

To identify the regions of AIMP1 which is cleaved by elastase 2, the N-terminal amino acid sequence of the elastase 2-cleaved AIMP1 fragments obtained in Test Example <1-1> were determined by N-terminal amino acid sequencing using the Edman degradation method and an automated sequence analyzer, and the cleaved sites of AIMP1 were identified (see FIG. 3).

According to the sequence information determined as described above, several deletion mutants of the AIMP1, i.e., AIMP1-(1-192), AIMP1-(6-192), AIMP1-(30-192), AIMP1-(47-192), AIMP1-(54-192), AIMP1-(101-192), AIMP1-(114-192), AIMP1-(1-46), AIMP1-(1-53) and AIMP1-(193-312) fragments, were constructed (see FIG. 4). Each of the fragments was amplified by PCR using AIMP1 cDNA (SEQ ID NO: 9) as a template with specific primer sets (Table 1). The PCR was performed in the following conditions: 95 °C for 2 min; 30 cycles of 95 °C for 30 sec, 56 °C for 30 sec and 72 °C for 1 min; and 72 °C for 5 min.

**Table 1: Primer sets used in construction of AIMP1 deletion fragments**

| Primers | | Sequence | SEQ ID NO |
|---|---|---|---|
| AIMP1-(1-192) | Sense | 5'-CGGAATTCAT GGCAAATAAT GATGCTGTTC TGAAG-3' | 10 |
| | Antisense | 5'-GTCTCGAGTT AGCCACTGAC AACTGTCCTT GG-3' | 11 |
| AIMP1-(6-192) | Sense | 5'-CGGAATTCGC TGTTCTGAAG AGACTGGAGC AG-3' | 12 |
| | Antisense | 5'-GTCTCGAGTT AGCCACTGAC AACTGTCCTT GG-3' | 13 |
| AIMP1-(30-192) | Sense | 5'-CGGAATTCTC TCTACTTAAG GAGAAAGCAA TTTTG-3' | 14 |
| | Antisense | 5'-GTCTCGAGTT AGCCACTGAC AACTGTCCTT GG-3' | 15 |
| AIMP1-(47-192) | Sense | 3'-CGGAATTCAA ACTTCGAGTT GAAAATGCTA AACTG-3' | 16 |
| | Antisense | 5'-GTCTCGAGTT AGCCACTGAC AACTGTCCTT GG-3' | 17 |
| AIMP1-(54-192) | Sense | 5'-CGGAATTCAA ACTGAAGAAA GAAATTGAAG AACTG-3' | 18 |
| | Antisense | 5'-GTCTCGAGTT AGCCACTGAC AACTGTCCTT GG-3' | 19 |
| AIMP1-(101-192) | Sense | 5'-CGGAATTCGC AGTAACAACC GTATCTTCTG G-3' | 20 |
| | Antisense | 5'-GTCTCGAGTT AGCCACTGAC AACTGTCCTT GG-3' | 21 |
| AIMP1-(114-192) | Sense | 5'-CGGAATTCAA AGGAGGAACA GGAGACGAAA AG-3' | 22 |
| | Antisense | 5'-GTCTCGAGTT AGCCACTGAC AACTGTCCTT GG-3' | 23 |
| AIMP1-(1-46) | Sense | 5'-CGGAATTCAT GGCAAATAAT GATGCTGTTC TGAAG-3' | 24 |
| | Antisense | 5'-GTCTCGAGTT ACTTCTCTTC CCTCAAAGTT GCC-3' | 25 |
| AIMP1-(1-53) | Sense | 5'-CGGAATTCAT GGCAAATAAT GATGCTGTTC TGAAG-3' | 26 |
| | Antisense | 5'-GTCTCGAGTT AAGCATTTTC AACTCGAAGT TTC-3' | 27 |
| AIMP1-(193-312) | Sense | 5'-CGGAATTCCT GGTGAATCAT GTTCCTCTTG AAC-3' | 28 |
| | Antisense | 5'-GTCTCGAGTT ATTTGATTCC ACTGTTGCTC ATG-3' | 29 |

The PCR products were digested with *Eco*RI and *Xho*I and ligated into pGEX4T3 vector (Amersham Biosciences) cut with the same enzymes. *E. Coli* BL21 (DE3) was transformed with the ligates and cultured to induce the expression of the peptides. Each of the peptides was expressed as a GST-tag fusion protein and purified with GSH agarose. To remove lipopolysaccharide, the protein solutions were dialyzed in pyrogen-free buffer (10 mM potassium phosphate buffer, pH 6.0, 100 mM NaCl). After the dialysis, each of the proteins was loaded onto polymyxin resin (Bio-Rad) pre-equilibrated with the same buffer and then incubated for 20 minutes, and eluted. Each of the purified peptides was analyzed on SDS-PAGE (see FIG. 5).

### Example 2: Identification of AIMP1 domain having activity of stimulating fibroblast proliferation

In order to examine the fibroblast proliferation activities of the recombinant proteins constructed in Example 1, foreskin fibroblast cells were treated with each of the recombinant proteins in the same manner as in Test Example <1-2>, and the proliferation of the fibroblast cells was examined.

From the test results, it could be found that the N-terminal region of the AIMP1 stimulated the proliferation of fibroblast cells. Specifically, AIMP1-(1-312) (SEQ ID NO: 8), AIMP1-(1-192) (SEQ ID NO: 5), AIMP1-(1-46) (SEQ ID NO: 2), AIMP1-(1-53) (SEQ ID NO: 3) and AIMP1-(6-192) (SEQ ID NO: 4) showed high proliferation-inducing activity, but AIMP1-(30-192), AIMP1-(47-192), AIMP1-(54-192), AIMP1-(101-192), AIMP1-(114-192) and AIMP1-(193-312) did not (see FIG. 6). These results suggest that the N-terminal region of AIMP1, especially AIMP1-(6-46), be a domain that induces the proliferation of fibroblast cells.

### Example 3: Fibroblast proliferation-stimulating activity of AIMP1-(6-46) fragment

To prove the supposition that AIMP1-(6-46) is a domain that induces the proliferation of fibroblast cells, an AIMP1-(6-46) fragment was synthesized and the fibroblast proliferation-stimulating activity thereof was examined.

### <3-1> Construction of AIMP1-(646) fragment

A peptide (SEQ ID NO: 1) corresponding to amino acids 6-46 of AIMP1 was synthesized and the effect of the peptide on fibroblast proliferation was analyzed. The AIMP1-(6-46) fragment was prepared by PCR using AIMP1 cDNA as a template with the following specific primer set (SEQ ID NO: 30 and SEQ ID NO: 31). The PCR was performed under the following conditions: 95 °C for 2 min; 30 cycles of 95 °C for 30 sec, 56 °C for 30 sec and 72 °C for 1 min; and 72 °C for 5 min.
Sense primer of AIMP1-(6-46): (SEQ ID NO: 30)
   5'- CGG AAT TCG CTG TTC TGA AGA GAC TGG AGC AG-3'
Antisense primer of AIMP1-(6-46): (SEQ ID NO: 31)
   5'-GTC TCG AGT TAC TTC TCT TCC CTC AA A GTT GCC TG-3'

The PCR amplification product was digested with EcoRI and XhoI and ligated into a pGEX4T3 vector (Amersham Biosciences) cut with the same enzymes. E. *Coli* BL21 (DE3) was transformed with the ligates and cultured to induce the expression of the peptide, followed by isolation and purification by the same manner as in Example 1.

### <3-2> Fibroblast proliferation-stimulating activity

Whether the AIMP1-(6-46) fragment synthesized in Example <3-1> stimulates the proliferation of fibroblast cells was examined in the same manner as in Test Example <1-2>. Herein, the AIMP1-(6-46) fragment was used at various concentrations (0, 50, 100 and 200 nM), and 100 nM of the full-length AIMP1 (AIMP1-(1-132)) was used as a control.

The test results showed that the AIMP1-(6-46) fragment induced the proliferation of fibroblast cells in a dose-dependent manner and had an activity almost similar to the full-length AIMP1 (AIMP1-(1-312)) at the same concentration (see FIG. 7).

### Example 4: Wound healing-promoting activities of deletion fragments of AIMP1

AIMP1-(14-34) (SEQ ID NO: 6) and AIMP1-(26-46) (SEQ ID NO: 7) fragments smaller than the AIMP1-(6-46) fragment synthesized in Example 3 were synthesized by a chemical synthetic method in Peptron (http://www.peptron.co.kr), and the wound healing-promoting activities thereof were analyzed in vivo. The analysis of the wound healing activities was performed using 8-week-old C57BL/6 mice. The mice were anesthetized with an intraperitoneal injection of 2.5% avertin (100 µl/10g), and then the back of each animal was shaved, followed by disinfecting the back skin with 70% alcohol. The back skin was marked with a 0.5 cm-diameter circle, and a skin and panniculus carnosus muscle was removed with scissors to induce a wound. The wounds were left uncovered without a dressing. One wound was generated per mouse and treated with 200 nM of each of AIMP1-(14-34), AIMP1-(26-46) and AIMP1-(6-46) fragments in PBS (phosphate-buffered saline) containing 20% glycerol, twice a day at 12-hour intervals until day 8 after wounding. A control group was treated only with PBS containing 20% glycerol. Then, wound closure was monitored daily using the Image-pro Plus software and was calculated as the percentage of the initial wound area.

From the test results, it could be found that the AIMP1-(14-34), AIMP1-(26-46) and AIMP1-(6-46) had the activities of promoting wound healing. Also, the wound healing-promoting activities of the fragments were all similar to each other (see FIG. 8).

### Industrial Applicability

As described above, the inventive peptide has the activity of promoting wound healing by stimulating the proliferation of fibroblast cells.

## Claims

1. A composition for use in the treatment of a wound, comprising:
(a) a peptide consisting of the amino acid sequence of SEQ ID NO:1-3 or 6-7;
(b) a peptide consisting of 21-41 contiguous amino acids of SEQ ID NO:1; or
(c) a peptide having at least 90% identity to a peptide that consists of the amino acid sequence of SEQ ID NO:1.

2. The composition for use of claim 1, wherein the wound is a contusion, bruise, non-healing traumatic wound, tissue disruption caused by irradiation, abrasion, gangrene, laceration, avulsion, penetrated wound, gunshot wound, cutting, burn, frostbite, skin ulcer, xeroderma, skin keratosis, breakage, rupture, dermatitis, smart caused by a dermatophyte, surgical wound, wound caused by a vascular disorder, corneal wound, sore including pressure sores and bed sores, diabetic skin erosion or other poor-circulation associated condition, chronic ulcer, suture site, spinal traumatic wound, gynecological wound, or a chemical wound.

3. The composition for use of claim 1, wherein the peptide consists of the amino acid sequence of SEQ ID NO:1.

4. The composition for use of any one of claims 1 to 3, comprising a pharmaceutically acceptable carrier.

5. The composition for use of any one of claims 1 to 4 wherein the wound is treated by stimulating the proliferation of fibroblast cells.

6. Use of a peptide according to any one of claims 1 to 3 in the manufacture of a medicament for the treatment of a wound.

## Patentansprüche

1. Zusammensetzung zur Verwendung für die Behandlung einer Wunde, umfassend:
(a) ein Peptid, bestehend aus der Aminosäuresequenz von SEQ ID NO:1-3 oder 6-7;
(b) ein Peptid, bestehend aus 21-41 zusammenhängenden Aminosäuren von SEQ ID NO:1; oder
(c) ein Peptid, das zu 90 % identisch mit einem Peptid ist, welches aus der Aminosäuresequenz von SEQ ID NO:1 besteht.

2. Zusammensetzung zur Verwendung nach Anspruch 1, wobei die Wunde eine Prellung, eine Quetschung, eine nicht heilende traumatische Wunde, eine Gewebezerstörung durch Bestrahlung, eine Abrasion (Abschürfung), ein Gangrän (Wundbrand), eine Lazeration (Einriss), ein Décollement (Hautabscherung), eine Stichwunde, eine Schusswunde, eine Schnittwunde, eine Verbrennung, eine Frostbeule, ein Hautgeschwür, Xerodermie (trockene Haut), Hautkeratose, eine Bruch-, Risswunde, Dermatitis, ein Brennen der Haut verursacht durch einen Dermatophyten (Hautpilz), eine chirurgische Wunde, eine Wunde verursacht durch Gefäßkrankheiten, eine Wunde der Augenhornhaut, eine wunde Stelle einschließlich Druckwunden und Dekubitus, eine diabetische Hautveränderung oder ein anderer mit schlechter Durchblutung assoziierter Zustand, ein chronisches Geschwür, eine Nahtstelle, eine spinale traumatische Wunde, eine gynäkologische Wunde oder eine chemische Wunde ist.

3. Zusammensetzung zur Verwendung nach Anspruch 1, wobei das Peptid aus der Aminosäuresequenz von SEQ ID NO:1 besteht.

4. Zusammensetzung zur Verwendung nach einem der Ansprüche 1 bis 3, umfassend einen pharmazeutisch anerkannten Trägerstoff.

5. Zusammensetzung nach einem der Ansprüche 1 bis 4, wobei die Wunde durch Stimulation der Proliferation von Fibroblastenzellen behandelt wird.

6. Verwendung eines Peptids nach einem der Ansprüche 1 bis 3 bei der Herstellung eines Medikaments zur Behandlung einer Wunde.

## Revendications

1. Composition pour une utilisation dans le traitement d'une plaie, comprenant :
(a) un peptide constitué de la séquence d'acides aminés de SEQ ID NO : 1 à 3 ou 6 ou 7 ;
(b) un peptide constitué de 21 à 41 acides aminés contigus de SEQ ID NO : 1 ; ou
(c) un peptide présentant au moins 90 % d'identité avec un peptide qui est constitué de la séquence d'acides aminés de SEQ ID NO : 1.

2. Composition pour une utilisation selon la revendication 1, où la plaie est une contusion, une ecchymose, une plaie traumatique ne cicatrisant pas, une lésion tissulaire provoquée par des irradiations, une abrasion, une gangrène, une lacération, une avulsion, une plaie profonde, une plaie par balle, une coupure, une brûlure, une engelure, un ulcère cutané, une xérodermie, une kératose cutanée, une déchirure, une rupture, une dermite, une sensation de brûlure provoquée par un dermatophyte, une plaie chirurgicale, une plaie provoquée par un trouble vasculaire, une plaie de la cornée, un escarre y compris des escarres de décubitus, une érosion cutanée diabétique ou une autre affection associée à une circulation médiocre, un ulcère chronique, un site de suture, une plaie traumatique spinale, une plaie gynécologique ou une plaie chimique.

3. Composition pour une utilisation selon la revendication 1, dans laquelle le peptide est constitué de la séquence d'acides aminés de SEQ ID NO : 1.

4. Composition pour une utilisation selon l'une quelconque des revendications 1 à 3, comprenant un support pharmaceutiquement acceptable.

5. Composition pour une utilisation selon l'une quelconque des revendications 1 à 4, où la plaie est traitée par stimulation de la prolifération des fibroblastes.

6. Utilisation d'un peptide selon l'une quelconque des revendications 1 à 3, dans la fabrication d'un médicament destiné au traitement d'une plaie.
